# EUROPEAN PATENT SPECIFICATION

(11) **EP 1 157 692 B1**
(45) Date of publication and mention of the grant of the patent: **05.10.2005**
(21) Application number: 00110811.7
(22) Date of filing: 22.05.2000
(51) Int. Cl.: A61P 9/00

(54) **Composition of fatty acids containing at least 80% by weight of EPA and DHA or their derivatives and its pharmaceutical use**
Fettsäure enthaltende pharmazeutische Zusammensetzung die wenigstens 80 Gew. % EPA und DHA enthält
Composition pharmaceutique d'acides gras, contenant au moins 80% en poids de EPA et DHA

(43) Date of publication of application: 28.11.2001
(73) Proprietor: PRO APARTS - INVESTIMENTOS E CONSULTORIA LDA, 9000-060 Funchal, Madeira (PT)
(72) Inventor: Shibuya, Hajime, 201-0004 Tokyo (JP)
(74) Representative: Mancini, Vincenzo

(56) References cited:
- US-A- 5 656 667

## Description

The present invention refers to a composition of fatty acids containing at least 80% by weight of 5, 8,11, 14, 17-eicosapentaenoic acid (EPA) and 4,7,10,13,16,19-docosaexaenoic acid (DHA) (double bonds all cis), or their derivatives , where the ratio between EPA and DHA is within 0.9 and 1.5 and other ω-3 C₂₀, C₂₁ and C₂₂ acids constitute less than 3 percent, and its pharmaceutical use.

In the 1970's, Bang, Dyerberg and other authors brought to the attention of the scientific community the potentially useful benefits of a diet containing a source of polyunsaturated fatty acids as the fish oils (H.O.Bang et al., Lancet 1, 1143,1971; J.Dyerberg et al., Am J. Clin.Nutr. 28, 958, 1975).

By studying a population of Greenland Eskimos , they found that while their diet was relatively rich of acids and poor of carbohydrates, the incidence of cardiovascular disease was very low in comparison with the other populations and with the scientific opinion of the time.
Among minor symptomatic findings it was found through serological tests that the Eskimos had plasma levels of total lipids, cholesterol, lipoproteins and triglycerides lower than those of Eskimos living in Denmark taken as reference group.

On the basis of deep analysis of the lipidic picture, it was thought that the differences could be attributed to the high levels of polyunsaturated ω-3 acids, deriving from the diet of Eskimos who in fact took high quantities of fats all or marine origin (fish oils), containing high percentages of such acids. It was also concluded that the differences could not be of genetic origin, because the Eskimos migrated in Denmark and brought to take a diet of a more western type, richer of saturated fats showed an incidence of cardiovascular illness more similar to that of Eskimos born and growth in Denmark (J.Dyerberg et al., Dan. Med.Bull. 24, 52, 1977; J.Dyerberg et al., Lancet 2, 117, 1978; ibid 2, 433, 1979).
As the cardiovascular pathology is the highest cause of mortality in all countries, as and more than the tumor illness, it is obvious that such findings could bring to an enormous number of studies and researches during the following decades, with the highest engagement of all the scientific and pharmaceutical world.

Further purpose of such studies has been that of verifying the range of the possible effects of ω-3 fatty acids, besides the control of the homeostasis, of the lipidic pattern and of the atherosclerosis (Bang, Dyerberg, etc., above mentioned), also on metabolically mediated diseases such as hypertension (R.Lorenz et al., Circulation 67, 504, 1983; J.Z.Mortensen et al., Thromb.Haemost,. 50, 543, 1983), tumor illness (R.A.Karamali et al., J.Nat.Cancer Inst. 75, 457, 1984), diabetes, rheumatoid arthritis, multiple sclerosis, psoriasis etc., as well as on the involved action mechanisms, usually related to the action of all C₂₀ eicosanoids and on their metabolites (prostaglandins, prostacyclines, thromboxanes, leukotrienes).

The results of such studies brought to numerous and important knowledges on the effects of ω-3 acids, but the researches in this field need certainly to be further deepened because most of such studies showed heavy faults as underlined in reviews of the available studies (H.R.Knapp et al., Proceeding of AOCS Short Course on polyunsaturated fatty acids and eicosanoids, editor W.E.M.Lands, pages 41-55, American Oil Chemists Society; K.BØnaa, Tidskr.Nor.Laegeforen N.28, 1987, 2425-8).

We think that unfortunately too many of such studies have been conducted on the "fish-oils" as such or simply refined or alternatively on the so-called ω-3 acids as they would have been single entities or "simple" mixtures or anyway well reproducible mixtures , also obliged to do so by the extreme difficulty of purification of such complex mixtures and/or of isolation of the single components all extremely similar among them as for their chemical and physical properties : all this has hindered - with just a few exceptions - to detect and define the effects of the single components.
For example, the effect of fish-oils in preventing atherosclerosis or hyperlipemia, claimed be Dyerberg et al. (see above), by Casali et al. J.Surg.Res. 40, 6, 1986; by Weiner et al., N.Engl.J.Med., 315, 841, 1986; by H.R.Davis et al., Artheriosclerosis 7, 441, 1987; has been then denied by J.Thiery et al., Atherosclerosis 63, 53, 1987.

Similarly, the effect on the coronary disease has been assessed by D.Kromhout et al., N.Engl.J.Med. 312, 1205, 1985 and by R.B.Shekelle et al., N.Engl.J.Med. 313, 820, 1985 and then denied by S.E.Vallset et al., N. Engl.J.Med, 313, 820, 1985 and by J.D.Curb et al., N. Engl.J.Med. 313, 821, 1985.

On the other hand, as evidenced also in the It.Pat. No. 1,235,879, also the eicosapentaenoic acid (EPA), which has been considered the most important of marine polyunsaturated acids belonging to the ω-3 series (see below) has been recognized free from any significant action on hypertension (in : Prostaglandins, Vol.32 (2), 179, 1986; Terano et al., Atherosclerosis 46, 321, 1983; Yoshida et al., Artery 14, 295, 1987; Yin et al., Clin.Exp.Pharm.Phys. 15, 275, 1988).

Several other incongruities could be reported.
In conclusion, many literature data , explained as if they were obtained from constant compositions, brought to contrasting conclusions and to great confusion.

Indeed, the composition of fish oil is very complex in terms of number and structure of the constituent fatty acids. A first classification divides such acids into saturated, mono-unsaturated and polyunsaturated acids and, inside to each class , according to the progressive number of carbon atoms (usually, from C₁₄ to C₂₂) and, in the case of polyunsaturated acids, according to the number of double bonds. In terms of specific acids, the composition of most of the marine species is constituted by at least 6-10 major fatty acids.

By using the convention X : Y ω : z (or n : z) where X is the number of carbon atoms of the acid chain, Y is the number of double bonds and z is the position number of the first carbon involved in a double bond starting from the end methyl (ω or n) of the chain, the most common fatty acids are C14 : 0, C16 : 0 among the saturated , C16 : 1 ω-6, C18 : 1 ω-6 among the monounsaturated, C20 : 5 ω-3 (eicosapentaenoic acid, EPA) and C_{22 : 6} ω-3 (docosaexaenoic acid, DHA) among the polyunsaturated acids (R.G.Ackman, Fatty acid composition in fish-oils. In: Nutritional Evaluation of Long Chain Fatty acids. S.M.Barlow and M.E.Standsby Ed., p.25-78, 1982. Academic Press, New York, N.Y.).

These components sum up frequently till 80-85% of the total, but in addition the literature reports many other minor acids, each in variable quantity from traces till a few percent, and in all up to about 50 and, anyway, always in the order of numerous tens, according to the marine species and other individual, environmental and biological. factors.

In this situation it is not surprising that various fish-oil preparations can give, probably in relation with the various batches, conflicting results (see literature above) or even opposite clinical effects (see effects on restenosis following coronary angioplasty : G.J.Dehmer et al., N.Engl.J.Med. 318, 733, 1988; L.E.Grigg et al., J.Am.Coll.Cardiol. 13, 665, 1989; G.J.Reis et al., Lancet, 2, 177, 1989).

Also the preparations constituted according to the common definition by "enriched" ω-3 acids constitute an unacceptable generalization because there is no demonstration that they are in some way biologically equivalent. On the contrary it is known and documented that even a small modification in the number or in the position of a single double bond or in the number of carbon atoms may condition in a definite way the biological activity of the substance or even may induce a quite opposite effect, all this outside of the same class of acids (for instance polyunsaturated vs monounsaturated vs saturated acids, or ω-3 vs ω-6 acids), but also inside the same class, for instance among the various ω-3 acids.

See for example the effects of arachidonic acid C_{20 : 4} ω-6, which by action of cyclo-oxygenase leads to eicosanoids endowed as a whole with vasoconstrictive and pro-aggregating activity, in opposition to the effects obtained with EPA C₂₀ : 5 ω-3, which differs for having only 1 double bond more (A.Bonanone et al., Trombosi & Aterosclerosi 1, 5, 1990); see DHA C₂₂ : 6 ω-3 which shows a very high tropism for some tissues (retina, testes, etc.) and particularly for the nervous system tissue (brain tissue, where it constitutes 20-40% of the phospholipidic content) and which is at present the basis of specific studies on relevant pathologies, such as dementia, learning defects, etc., a picture quite different from that of EPA C₂₀ : 5 ω-3 (A.Leaf et al., N.Engl.J.Med. 318. 549, 1988; M.Neuringer et al., Nutr.Rev. 44, 285, 1986); see linoleic acid C18 : 2 ω-6 which is able to induce a significant decrease of cholesterolemia, differently from other acids of the ω-6 series (M.S.Brown et al., Science 232, 34, 1986; J.Shepherd et al., J.Lipid Res. 21, 91, 1980); etc.

To conclude, with the exception of relatively few studies carried out with sufficiently definite compositions of polyunsaturated acids, related to the major components of fish oils and - since more abundant - more easily isolated like EPA and DHA, it is not possible to be sure of the results obtained and then of the real efficacy of such mixtures and not even of the reproducibility of their effect.

Also the generalized mentioning of ω-3 acids and the assessments on their global biological/clinical effects - as though all the components of the class should be equivalent - appear to us quite unacceptable and must be re-evaluated again.

A typical example of unjustified generalisation is represented by the It.Pat. 1,235,879. This patent claims a composition of fatty acids comprising at least 80% by weight of ω-3 polyunsaturated acids where EPA and DHA - in the ratio from 1 : 2 to 2 : 1, that is between 0.5 and 2 - constitute at least 75% and other ω-3 acids C₂₀, C₂₁ and C₂₂ constitute at least 3%. Other claims refer to compositions comprising at least 90% (or 95%) of ω-3 acids where EPA and DHA constitute at least 85% (or respect. 90%), while other ω-3 acids C₂₀,C₂₁ and C₂₂ constitute at least 4.5% by weight. We believe that such claims, as expressed, are quite ambiguous because they lead to think that other ω-3 acids C₂₀, C₂₁ and C₂₂ are endowed with the same activity of EPA and DHA which is not absolutely demonstrated as they have not ever been isolated as pure products nor tested for their biological, pharmacological and clinical effects and lead then to think that they give a relevant contribution to the claimed composition and that, at the end, their presence, in quantity at least of 3% or better of 4.5% or more, is an advantage as far as purity and efficacy of the composition are concerned.

On the contrary, we think that such minor components must be thought for all purposes as impurities and that, being already present in the natural fish oils, their presence in the claimed concentrated compositions is essentially caused by isolation difficulties because they are long chained acids C₂₀, C₂₁ and C₂₂ and highly unsaturated compounds (C_{20 : 4} ω-3, C_{21:5} ω-3, C_{22:5} ω-3) which differ very little in all the chemical-physical parameters from the required EPA (C_{20:5} ω-3) and DHA (C_{22:6} ω-3). It can be read in fact in said patent, page 10, line 4 and following : other ω-3 acids of the series C₂₀, C₂₁ and C₂₂ will be obtained more or less in their original concentrations, for instance 3-5% by weight, typically at least 4.5%. Then, the particular acid C_{21:5} ω-3 is normally present in concentrations of at least 1.5% by weight and the acid C_{22 :5} ω-3 normally in concentration of about 3.0% by weight.

All that specified, we have thought, found and studied a composition limited in a much more severe way and, therefore, much more reproducible in its components and in its effects, that is a composition of fatty acids comprising at least 80% by weight of EPA and DHA (or their derivatives), where the ratio EPA to DHA is within 0.9 and 1.5, preferably within 1 and 1.4, and other ω-3 acids C₂₀, C₂₁ and C₂₂ are less than 3%.

Compositions particularly preferred are those containing at least 85% or at least 90% or at least 95% by weight of EPA and DHA, always with ratio and total content of ω-3 acids C₂₀, C₂₁ and C₂₂ above specified. The derivatives of EPA and DHA are represented by C₁-C₃ esters, preferably the ethyl esters, or additionally the salts with bases acceptable from the pharmaceutical point of view.

By using the above specified composition, we have first found a total reproducibility of biological effects and have further demonstrated their efficacy against slight and moderate cases of hypertriglyceridemia, hypercholesterolemia (with decrease of VLDL and increase of HDL values), systolic and diastolic hypertension, and of hyperactivity of the factor VII of the blood coagulation, that is against the so-called cardiovascular risk factors; above all, we have shown a very high efficacy in the clearest forms of hyperlipidemia and hypertension, being on the contrary such compositions quite ineffective in normal situations with no modification of the related parameters under the physiological values.

By using the same compositions we have also found that they were able to hinder several forms of heart pathologies such as arrhythmia, atrial and /or ventricular heart fibrillation and - for the particular seriousness of these pathologies - also this result represents a primary object of the present invention.

Together with the heart deficiency (pump failure , of mechanical origin), the cardiac problems of electrical type as arrhythmia and fibrillation are the principal causes of death in cardiopathic subjects, both primarily and as a secondary complication post-infarction : it is then obvious the interest for the preventive and therapeutic use of the compositions under discussion.

It is then consequential the therapeutic indication in the primary and particularly in the secondary prevention, in the patients with previous myocardial infarction, of the complications in the cardiopathic patient, to overcome the problems deriving from electric hyperexcitability , from heavy disorders in the diffusion of excitement and from other problems with the threshold of electric polarization of myocardial cells (arrhythmia, fibrillation) and to reduce the risk of mortality, particularly the so-called "sudden death" caused above all by the sudden and fatal onset of crisis of ventricular fibrillation.

With reference to the composition of fatty acids according to the present invention, it is obtained according to the general procedures substantially already known in the literature, but obviously using some precautions absolutely essential to the requested purpose. First of all, the fish oil is submitted to hydrolytic procedures, for instance by means of alcoholic potassium hydroxide and in mild experimental conditions to avoid damages to the delicate structure of the polyunsaturated components (heating at moderate temperature, presence of antioxidants, inert atmosphere, etc.). An alternative to the use of potassium hydroxide could be the use of lithium hydroxide which leads to the corresponding salts of the fatty acids, of which the saturated or less unsaturated components are less soluble and can be filtered off, thus permitting a first fractioning and enrichment of the mixture of polyunsaturated fatty acids.

While these procedures lead directly, through the corresponding salts, to the fatty acids, the composition of the invention constituted by the esters, particularly by the ethyl esters, are obtained by direct esterification eventually at the end of the process for obtaining the concentrated composition, or preferably it is possible to proceed with the direct transesterification of fish oils (instead of the hydrolysis), which is carried out in the presence of the suitable alkanol C₁-C₃, preferably of ethanol, and of an alkaline catalyst, for instance potassium hydroxide itself, or even of an acid catalyst like H₂SO₄.

The mixture of fatty acids or of the corresponding esters is then made to complex with urea, such reaction being preferably carried out in ethanol or even in methanol. It is known that urea , under particular conditions, crystallizes in hexagonal crystals by forming channels able to include the straight chains of fatty acids. It is also known that urea is able to include and to complex essentially the saturated and/or monounsaturated acids and esters, leading to precipitation of the complex which is then filtered off which permit then to fractionate the mixture and to recover from the solution a composition strongly enriched in polyunsaturated components, like EPA and DHA.

A third essential phase for obtaining a composition strongly enriched in EPA and DHA, frequently but not necessarily to be carried out as final step of the procedure, consists in a distillation of the mixture which may be done accordingly to all techniques known to the expert, but always taking care of working at reduced temperature and pressure so as to avoid even partial degradations (isomerization, polymerisation, etc.) of the polyunsaturated structures.

One of the procedures often adopted is the molecular distillation, which permits to carry out the distillation on a thin layer of oily material and to avoid, by means of a continuous supply of the material, a prolonged heating of the whole mass of substance.

Useless to say that the whole of these procedures allows also the purification of the fish oil and of the related fatty acids from the usual polluting substances present in crude oils, of marine origin or not (unsaponifiable matter, cholesterol, pesticides, heavy metals, chlorinate hydrocarbons etc.). A procedure in accordance with what above reported is that described in the Italian Patent No. 1,205,043.

We have however found that it is quite necessary, to obtain the composition here claimed, to take several precautions : without them it would be impossible to reach the desired concentration and ratios of EPA and DHA and to limit the other ω-3 acids C₂₀, C₂₁ and C₂₂ under 3%. These last would be otherwise increased respect to the starting composition and respect to such 3% limit concentration (see in fact the mentioned It.Pat, 1,235,879 : page 12, lines 10-11 " such procedure not only increases the single acids ..."; see also the relevant values of Table 1 and Table 2).

We select fish oils in order that the composition of ω-3 acids C₂₀,C₂₁ and C₂₂ of the starting material be close to and possibly lower than the values desired for the final composition, their total content being usually lower than 4% and preferably lower than 3%.

The most abundant acid is often C_{22 : 5} ω-3 (< 2.5%), followed by C_{21 :} ₅ ω-3 and C_{20 : 4} ω-3 (<1% each) and others in traces.

It is known that the composition of oils depends both on the marine species and on several other factors and that it may vary according to environmental and biological causes.

Two main factors are diet of the fish and the normal metabolism : both are important in the synthesis of the saturated (20-30% of the total) and of the shorter monounsaturated acids, while the monounsaturated C_{20:1} and C₂₂ : 1 and the long chain polyunsaturated acids are mainly of dietary origin. Chain elongation and desaturation mechanisms play a minor role.

As the food assumption changes according to the reproductive or migratory cycles, the diet may widely vary the composition of depot fats. Similarly, the variation according to annual cycles of the water temperatures, of the currents etc., conditions the composition and the availability in fatty acids of the phytoplankton of invertebrates and of crustacea and then of the marine species directly feeding on, as well as of the predatory species. Great variability is due also to the fishing area and to the type of water-culture production. Depending on these and other parameters, the oils of a marine species may changes up to 5-10 times the fatty acids present in lower quantity and up to 2-3 times those in higher quantity. It is therefore necessary a strict monitoring from the producer, with analyses severe and done on big batches of the extracted oils, with the purpose to obtain sufficiently constant mean composition. In spite of the difficulties, through a careful control it is possible to obtain a fish oil according to the above defined specifications.

An oil often in line with the requests is the herring oil, but of course this is not strictly needed and is just mentioned as an example.

We have found that the use of some amount of urea in excess to the quantity usually employed and reported in literature to eliminate the saturated and monounsaturated components and to come to a mixture enriched in polyunsaturated acids (3 parts by weight respect to the mixture of acids or esters), for instance the use up to 1-2 further parts by weight, for instance divided in 1-3 additional phases of complexation, aids the inclusion and the elimination of a part of ω-3 acids C₂₀, C₂₁ and particularly C₂₂, reducing their presence in the concentrated composition. In other words, while it is usually recognized that urea is specific in complexing the monounsaturated and saturated acids, with the result that all the other polyunsaturated components increase their concentration (both EPA and DHA and the other above mentioned minor components), we have found and adopted the finding that this property is not absolute. The excess urea is therefore able to include also the acids with higher degree of unsaturation, even though partially and with progressively growing difficulty, from the di/trienoic to the tetra/pentaenoic acids : by using a pre-enriched mixture of polyunsaturated acids, it is possible to obtain a significant reduction of ω-3 acids C₂₀, C₂₁ and C₂₂ with lower unsaturation than EPA and DHA. The yield of EPA and DHA is only slightly decreased , because they also are moderately involved in the process of complexation and inclusion in the urea. Such 1-3 further treatments with additional limited quantities of urea (till 1-2 parts by weight) are carried out in the usual solvents, as ethanol or methanol and also - should the urea be already in the necessary hexagonal crystallographic form - even without any solvent.

To the purpose of reaching the specifications above defined, we have also used a further innovation : we have found that the acids and the esters endowed with the higher degree of unsaturation have boiling points just a little lower than those of the components less unsaturated of same length and, in particular, than the saturated ones.

The difference of boiling points, already important to fractionate very complex mixtures and to enrich them in components with longer chains and higher molecular weights, has been found useful by us to lower the content of less unsaturated components in comparison with those more unsaturated, for instance of C_{22 5} ω-3 in comparison with C_{22:6} ω-3 (DHA) and of C_{20:4} ω-3 in comparison with C_{20:5} ω-3 (EPA), so that as a whole the ω-3 compounds C₂₀, C₂₁ and C₂₂ resulted to be < 3%. Also the corresponding acids/esters of the ω-6 series resulted to have reduced content.

Such procedure will be generally adopted with the molecular distillation technique, that is by using very high vacuum, relatively low temperature and continuous feeding, quite reducing any degradation process of the polyunsaturated structures caused by extreme and prolonged heating and, possibly, by using a double stage equipment which permits in just one step the separation of eventual lower boiling "heads" of distillation and higher boiling undistilled "tails".

With reference to the use of the compositions of the invention, we have found that their effectiveness is similar to that reported in the mentioned It.Patent n. 1,235,879 which referred particularly to slight hypertension (~150 mm Hg) and to hypertriglyceridemia in general, see page 7 line 16 (> 2 mMoles/l, little higher than 200 mg/dl), but it is above all evident in the typical forms, of familial and genetic origin, of hypertriglyceridemia and hypertension. Also such pathologies may be defined , as in the above patent, cardiovascular risk factors, however it is obvious that the finding obtained with our composition is unexpected and endowed with quite higher therapeutic and practical value. The studies with our composition have been particularly deepened in subjects with hypertriglyceridemia higher than 1000 mg/dl, or with systolic pressure higher than 170 mmHg and with 1 month treatments with 3 soft gelatine capsules daily, each capsule containing 1 g of the composition under study. This reduced posology in comparison to that adopted in the mentioned It.Pat. 1,235,879 (6 g daily) is very important to guarantee a high compliance of the patient respect to the intake of higher quantities of oily substances endowed with unpleasant organoleptic properties (smell and taste of fish and going rancid tendency) and therefore disagreeable to the patient.

It is noteworthy the reduction of cost of therapy, often limiting the reimbursability of the drugs by the National Health Services. The therapeutic results, particularly the reduction of hypertriglyceridemia are quite exceptional, while never showing a reduction of blood levels under those considered physiological.

The most important effect obtained with the composition under study has been anyway that of inhibiting harmful situations of cardiac arrhythmia, caused by electric hyperexcitability of myocytes and to heavy disorders in the diffusion of the excitement and of the electric conduction provoked at their turn by various factors, above all situation of local hypoxia or anoxia induced by coronary block and by infarction status. To carry out quick and highly reproducible controls it has been preferred the *in vivo* experimental technique on various animal species, particularly in small size animals like mouse and rat : it has been therefore possible to make a wide investigation without risks for the patient and by using a high number of animals so as to permit a careful statistical evaluation.

Techniques based on the visual observations of the animals to check the electrophysiological answer of myocardium have been avoided as thought too subjective. An adopted method used aconitine as arrhythmogenic agent and ECG as measure system, by determining the time necessary for the onset of initial cardiac arrhythmia and ventricular tachycardia. The animals were treated with the composition under study and with the control solutions before inducing the arrhythmia with aconitine to verify the preventive action and after arrhythmia to verify the therapeutic effect. In experiments carried out with higher doses of aconitine, usually the lower dose inducing 100% mortality of animals because of the occurred cardiac effects, it has also been possible to demonstrate the effectiveness of our composition in preventing the animal death and/or in prolonging its survival through a stabilization of myocyte membrane and of their electric excitability and through a reduction of the other complications induced by the arrhythmic status.

Another adopted method consisted in provoking arrhythmia and ventricular fibrillation in the anaesthetized rat by inducing an ischemic status by means of coronary ligation without any administration of arrhythmogenic agents. The composition under study resulted effective also in preventing arrhythmias deriving from coronary reperfusion.

In all these experiments the composition was administered both by parenteral and by oral route but for the clinical use also any other route of administration which can guarantee a systemic absorption may be adopted, including the intravenous, intramuscular, subcutaneous, rectal, vaginal routes, etc. and eventually also the topical route. In the case of the parenteral administration, the composition of fatty acids can be used as such or in the form of esters, preferably by slow infusion and in emulsified and stabilized formulation, or in the form of hydrosoluble salt and, at any rate, maintaining the necessary precautions common to the other lipid preparations, obviously always needed in the clinical use. The clinical administration by intravenous or intramuscular route is particularly useful for an acute therapy because of the promptness of its answer. The injectable pharmaceutical forms comprise phials or vials containing 0.25-5 g of active ingredient, until to bottles for slow infusion containing 5-10 g or more in 100 , 250 and 500 ml of aqueous/emulsifying solvent in sterile form. Because of its high tolerability, the dosage is never a limiting factor. The other foreseen formulations are prepared according to the praxis usual in the pharmaceutical technique.

In any case, the route of administration preferred for chronic or subchronic use is the oral route for which the pharmaceutical formulation can be prepared with techniques and excipients conventionally adopted for active ingredients which are in the oily form like those described for instance in Remington's Pharmaceutical Sciences Handbook, Hack Publ.Co., N.Y., USA. For the oral use, the preferred formulation for the oily substance is that in soft capsules, particularly soft gelatine, but also hard capsules and tablets on solid excipient, emulsions, granules in dispersing excipients, drops, syrups, etc. can be used. In the oral practice, the unitary dose comprises generally 250-1500 mg of active ingredient, preferably 500-1000 mg, and the daily dose is usually around 0.5-5 g or more preferably 1-3 g. With the oral administration it is also useful to evidence that the therapeutic answer is often relatively slower, depending on the absorption time, when the formulation contains the free fatty acids or their salts, biologically active as such; if, on the contrary, they are used in the form of esters, particularly of ethyl esters, the answer is even slower because the ester is per se just a little or no effective at all and its effectiveness is linked then to its metabolization time to give the relevant acid. The administration is then even more conditioned by the subchronic or, anyway, by the repeated use, so as to saturate the lipidic pool of any district with physiological derivatives (acids, glycerides, phospholipids, etc.) particularly the cardiac district and the cells of myocardium.

Still with reference to the various pharmaceutical formulations, they can obviously comprise , beside the composition of the invention and other drugs in combination having complementary and/or synergic activity, also one or more vehicles pharmaceutically acceptable well known in the technique as well as diluents, binders, stabilizers, surfactants, lubricants and similar, as usual and known in the pharmaceutical technique. Highly preferred, considering the polyunsaturated character of the active composition, is the presence of antioxidant agents like vitamin E (tocoferol), ascorbyl palmitate, ascorbic acid, hydroxytoluene and similar agents, as well as the presence of preservatives, colouring matters, flavours, perfumes and other pharmaceutical agents. Such formulations, beside the pharmaceutical use, can be destined also to the use as dietetics or alimentary integrators. The object of the present invention is better illustrated by some examples.

### Example 1

A. For comparison purpose, a 1 kg sample of a mixture of fish oils having the composition reported in Table 1 has been treated under nitrogen with boiling ethanol and in the presence of conc sulfuric acid, according to Example 1 of US Pat.5,130,061. After transesterification and following distillation of the ethanol, extraction with hexane, evaporation under vacuum of the solvent and double stage molecular distillation, followed by urea treatment essentially in agreement with the process of Example 8, an oil has been obtained constituted (Table 1) by : EPA+DHA (ethyl esters) >80% (81.3%), ratio < 0.9 (0.77, decreased in comparison to the starting oil) and sum of the other ω-3 fatty acids (ethyl esters) C₂₀, C₂₁ and C₂₂ >> 3.0% (7.6%).
B. For comparison purpose, a 1 kg sample of a mixture of fish oils having composition reported in Table 2 has been transesterified with ethanol and basic catalyst to give the corresponding ethyl esters; these last have been concentrated by complexing with the urea and the concentrate has been submitted to molecular distillation, as claimed (but not described) in claims 14 and 15 of It.Pat. 1,235,879, essentially in agreement with known procedures of the literature.
It has been then obtained an oil constituted (Table 2) by : EPA+DHA (ethyl esters) > 80% (82.4%), ratio >1.5 (1.65) and sum of the other ω-3 fatty acids (ethyl esters) C₂₀, C₂₁ and C₂₂ >>3.0 % (7.1%).

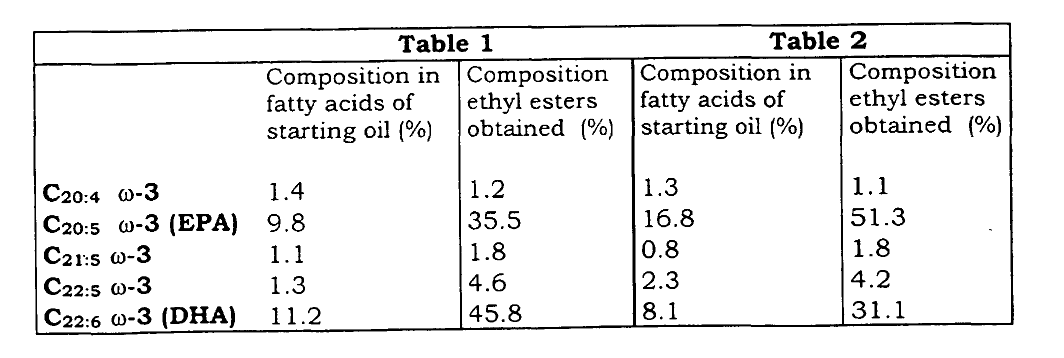

### Example 2

### A. Influence of the composition of starting oil

It is used an oil (in the case, herring oil), characterized by a relatively high content of EPA (18.2%) and DHA (11.8%) and by a limited content of other ω-3 acids C₂₀, C₂₁ and C₂₂, their sum being 3.9% as reported in Table 3.

1 kg of oil is treated with 2 l of ethanol in the presence of 12 g of potassium hydroxide (or even sodium ethylate) and kept under stirring for 2 hours under nitrogen, until the transesterification of glycerides to ethyl esters is complete. The reaction mixture is used as such or alternatively is made acid at pH 5-6 with 1M sulphuric acid and diluted with hexane, then the aqueous alcoholic phase is discharged and the organic phase is washed with water , dried and evaporated to dryness under vacuum.

The resulting mixture of ethyl esters is then added with a solution of 3 kg of urea in 18 l of boiling ethanol, then cooled at room temperature and maintained under slow stirring overnight at 5-10°. The abundant precipitate constituted by the complex of urea with the esters of saturated and less unsaturated acids, is filtered off, the solvent is distilled at reduced pressure, the residue is dissolved with hexane, washed with water, dried and brought to dryness.

Following molecular distillation under the usual conditions, an oil is obtained constituted (Table 3) by EPA+DHA (ethyl esters) >80% (82.7%), ratio < 1.5 (1.26) and sum of the other ω-3 fatty acids (ethyl esters) C₂₀, C₂₁ and C₂₂ at about 4-5% (4.8%).

### B. Influence of treatment with urea

It is used a fish oil (Table 4) transesterified in ethanol solution, for instance in agreement with Example 2A, but the treatment with urea is divided into 3 steps, respectively with 2, 1 and 0.5 parts by weight respect to the crude ethyl esters.

1 kg of crude ethyl esters is added to a mixture of 2 kg of urea in 12 l of boiling ethanol and under nitrogen then the mixture is cooled at room temperature and then at 0-10°C, under slow stirring overnight. The precipitated complex with urea is filtered and treated in succession with 1 kg of urea according to the same process and then, after additional filtration, again with 0.5 kg of urea. After filtration of the new complex and treatment according to standard methods, the mixture is evaporated to dryness at reduced temperature and pressure and the residue is submitted to molecular distillation. At the end an oil is obtained, constituted (Table 4) by EPA+DHA (ethyl esters) > 85% (85.3%), ratio < 1.4 (1.29) and sum of the other ω-3 fatty acids (ethyl esters) C₂₀, C₂₁ and C₂₂ <3% (2.7%).

### C. Influence of the molecular distillation

It is used a sample of ethyl esters (Table 5) obtained with the process of example 2B. The product is distilled with double stage molecular distillation, discarding a small lower boiling fraction and collecting the main fraction at 83-85° / 10⁻⁴ mm, the higher boiling fraction remaining as undistilled fraction. With gas-chromatographic analysis the product results to be constituted (Table 5) by EPA+DHA (ethyl esters) >85% (88.3%), ratio < 1.4 (1.32) and sum of the other ω-3 fatty acids (ethyl esters) C₂₀, C₂₁ and C₂₂ < 3% (2.2%).

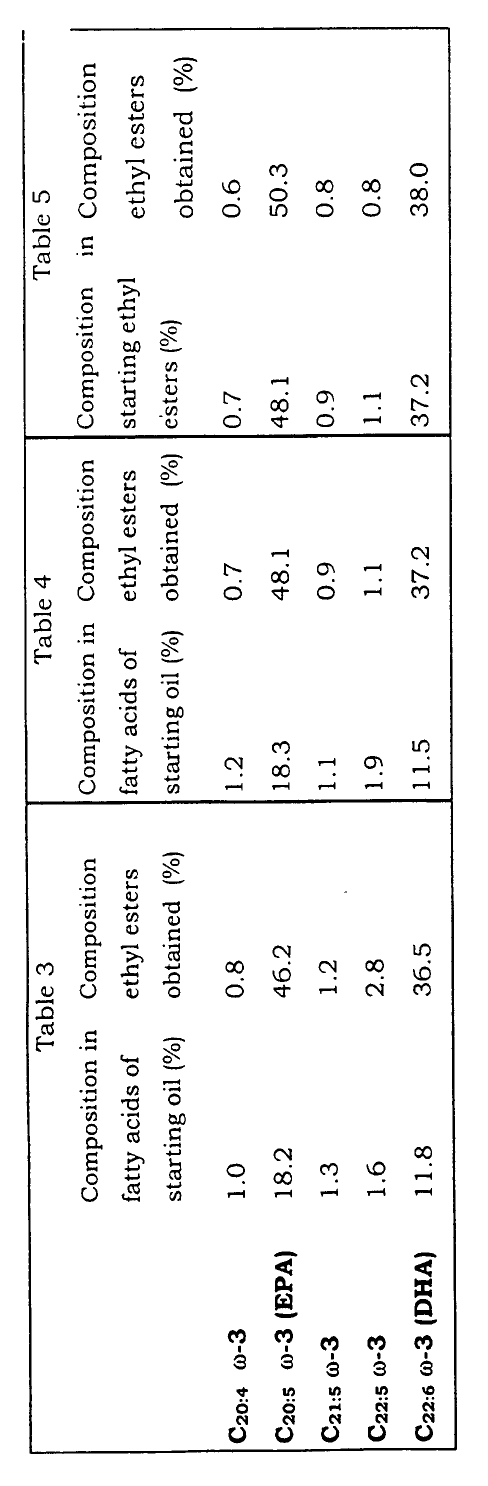

### Example 3

A fish oil with high content in EPA and DHA and reduced content in other ω-3 acids C₂₀, C₂₁ and C₂₂ is used, repeated phases of concentration with urea are carried out and molecular distillation in controlled condition of vacuum and temperature is adopted.

1 kg of fish oil with the composition reported in Table 6 is submitted in succession to the processes shown in example 2A, 2B and 2C.
It is obtained at the end an oil constituted by ethyl esters of polyunsaturated fatty acids as represented in the same Table 6, that is by EPA+ DHA (ethyl esters) >> 85% (90.1%), ratio EPA/DHA >1 and < 1.4 (1.22) and sum of the other ω-3 ethyl esters of polyunsaturated fatty acids C₂₀,C₂₁ and C₂₂ < 3% (2.2%).

This kind of oils or similar have been used for all the biological trials.

**Table 6**

| Fatty acid | Composition in fatty acids of the starting oil (%) | Composition ethyl esters obtained (%) |
|---|---|---|
| C _{14:0} | 8.7 | 0.0 |
| C _{16 : 0} | 17.6 | 0.0 |
| C _{16:1} ω7 | 6.3 | 0.0 |
| 7-Me C ^{16:0} | 0.3 | 0.0 |
| C _{16:2} ω6 | 0.6 | 0.0 |
| C _{16:2} ω4 | 0.9 | 0.0 |
| C _{16:3} ω4 | 0.4 | 0.0 |
| C _{16:4} ω1 | 1.3 | 0.0 |
| C _{18:0} | 3.2 | 0.0 |
| C _{18:1} ω9 | 8.5 | 0.0 |
| C _{18:1} ω7/ ω5 | 2.6 | 0.0 |
| C _{18:2} ω6 | 1.1 | 0.0 |
| C _{18:2} ω4 | 0.3 | 0.0 |
| C _{18:3} ω6 | 0.2 | 0.1 |
| C _{18:3} ω3 | 0.8 | 0.1 |
| C _{18:4} ω3 | 1.7 | 2.5 |
| C _{18:4} ω1 | 0.1 | 0.4 |
| C _{20:1} ω9/ω7 | 5.5 | 1.6 |
| C _{20:1} | 0.1 | 0.0 |
| C _{20:2} ω6 | 0.2 | 0.0 |
| C _{20:3} ω6 | 0.1 | 0.4 |
| C _{20:4} ω6 | 0.5 | 0.9 |
| C _{20:4} ω3 | 1.1 | 0.7 |
| C _{20:5} ω3 (EPA) | 17.8 | 49.6 |
| C _{21:5} ω3 | 1.0 | 0.7 |
| C _{22:1} ω11/ω9 | 4.2 | 0.5 |
| C _{22:2} ω6 | 0.7 | 0.0 |
| C _{22: 4} ω6 | 0.1 | 0.0 |
| C _{22: 5} ω6 | 0.1 | 0.4 |
| C _{22:5} ω3 | 1.6 | 0.8 |
| C _{22: 6} ω3 (DHA) | 12.1 | 40.5 |
| Others | 0.3 | 0.2 |
| | | |
| Sum EPA + DHA | 29.9 | 90.1 |
| | | |
| EPA : DHA | 1.47 | 1.22 |
| | | |
| Other ω3 (C₂₀,C₂₁,C₂₂) | 3.7 | 2.2 |

### Example 4

To evidence the ability of the composition of the oils under study to lower the blood levels of triglycerides and of cholesterol it has been thought simpler to use a subacute experimental model consisting in pretreating the laboratory animals with such composition and in administering then a substance able, in the animal not treated with the drug, to increase such blood levels.

4 groups of rats of initial mean weight of 235 g ± 10 have been included in the experiment : groups A and B were used as reference, groups C and D were treated orally with 50 mg/kg and 100 mg/kg respectively for 7 days with the composition of oils obtained according to Example 2C (Table 5).

At the end groups B, C and D were treated with Triton WR1339 and, after fasting overnight, all groups were sacrificed and the blood was drawn and dosed for triglycerides and total cholesterol.

| | Triglycerides (mg/dl) | Total cholesterol (mg/dl) |
|---|---|---|
| Group A | 86 ± 14 | 53 ± 5 |
| Group B | 240 ± 35 | 182 ± 34 |
| Group C | 165 ± 23 | 106 ± 12 |
| Group D | 123 ± 41 | 73 ± 14 |

As experimentally known, Triton is able to increase clearly the level of triglycerides and cholesterol while the composition of ethyl esters under study was able to lower them towards physiological levels inducing a decrease of mean values of noticeable entity and statistically significant (P < 0.05) respect to the positive controls (group B).

### Example 5

The effect on severe forms of hyperlipemia in subjects having increased risk of a quick development of atherosclerosis and other cardiological pathologies has been verified by using soft gelatine capsules containing 1 g of the composition of Example 3 (Table 6).

The study has been conducted on 6 subjects with long-standing, severe hyperlipoproteinemia of type V, by treating them for 1 month with 3 capsules daily. The control group was constituted by 6 subjects with similar haematological parameters recently diagnosed who were followed for the same period with the only prescription of a low caloric and lipidic content diet .

The results are reported in the following table (lipidic content in mg/dl) :

| Treatment | Triglycerides | Total cholesterol | VLDL | LDL | HDL |
|---|---|---|---|---|---|
| Low lipidic content diet | 1343 ± 530 | 383 ± 78 | 236 ± 85 | 88 ± 43 | 33 ± 8 |
| 3 capsules/day | 475 ± 136 | 212 ± 54 | 93 ± 12 | 112 ± 24 | 38 ± 7 |
| (VLDL = very low density lipoproteins; LDL = low density lipoproteins; | | | | | |
| HDL = high density lipoproteins) | | | | | |

The above data clearly demonstrate a very high drop of triglyceride levels as well as a significant reduction of total cholesterol and a tendency to normalization of the lipoproteic picture.

### Example 6

The experiment has been conducted to evidence the effect of the composition obtained according to Example 2C (Table 5), in the prevention of mortality induced by quick administration of an arrhythmogenic agent (aconitine) in the rat.

Several groups of 10 male rats weighing 220-250 g were used to determine the minimum lethal dose for 100% animals following intravenous administration of aconitine in the conscious, not anaesthetized rat.

Aconitine induced a quick and direct effect on myocardium with quick onset of arrhythmia and fibrillation followed, at some doses, by death of the animal within 20 minutes by the injection. The arrhythmia could be followed with ECG recording, as well as, in preliminary experiments, by means of visual observation of heart (open chest). Such lethal dose resulted to be 0.1 mg/kg i.v.

Afterwards, another 3 groups of 10 rats were treated by oral route with 50, 75, 100 mg/kg of the composition under study for 7 days, while a further group was maintained as control. Two hours after the last administration, all animals were injected i.v. with 0.1 mg/kg aconitine, with the results above reported.

| | Control | 50 mg/kg | 75 mg/kg | 100 mg/ kg |
|---|---|---|---|---|
| Animal survival | 0/10 | 2/10 | 5/10 | 6/10 |

The composition under study was therefore able to prevent the mortality induced by an arrhythmogenic agent with an effective dose on 50% of animals (ED 50) in the order of 75 mg/kg or a little higher.

### Example 7

The experiment wanted to verify the acute effect of a composition of ethyl esters of fatty acids according to Table 5 and of the corresponding potassium salts, in the prevention of the sudden death induced by an arrhythmogenic agent (aconitine) in the rat. The potassium salts were obtained by mild treatment of the esters with stoichiometric KOH in water : ethanol 8 : 2 according to standard methods.

4 groups of 10 rats were treated with a single oral administration of 50 and 100 mg/kg of ethyl esters and with a single i.v. injection of 50 and 100 mg/kg of potassium salt, while a further group was kept as control. 1 hour after the administration, all the animals received aconitine (0.1 mg/kg, i.v.), obtaining the following answers :

| | Control | 50 mg/kg p.o. | 100 mg/kg, p.o. | 50 mg/kg, i.v. | 100 mg/kg, i.v. |
|---|---|---|---|---|---|
| Animal survival | 0/10 | 0/10 | 1/10 | 3/10 | 7/10 |

It is therefore evident that the ethyl esters are a little or no effective as such and show some latency of the effect so as to be metabolized in vivo into the corresponding active form of acids. As such, the esters result to be more useful to a continuous oral administration and to a chronic therapy than as anti-arrhythmic agents of prompt effect in emergency. The acids under the form, for instance, of hydrosoluble potassium salts, are anti-arrhythmic agents potent and able to prevent the mortality induced by arrhythmogenic agents.

### Example 8

To evaluate the anti-arrhythmic activity of the composition of Table 5, administered as prophylactic drug, it has been adopted a technique which - by ECG monitoring - permitted to evaluate the prolongation of the onset-time to aconitine-induced initial arrhythmia and then to ventricular tachycardia in mice.

6 groups of male mice were treated daily for 1 month respectively with oral and intraperitoneal physiological saline and with the composition under study, 50 and 150 mg/kg orally and 50-150 mg/kg intraperitoneally.

1 hour after the end of the last treatment the animals of all groups were anaesthetized with sodium pentobarbital (50 mg/kg i.p.) and each animal individually was placed on a surgical board on its dorsal side and treated by infusion through the tail vein with aconitine (5 mcg/ml) with a flow rate of 0.25 ml/min. The aconitine induced arrhythmia was detected and measured by ECG by using a physiograph equipped with electrodes and accessories useful to the ECG recording. The onset time of initial cardiac arrhythmia was taken at the time of the first discernable sign of persistent deviation (> than 5 seconds) from the normal sinus rhythm ; with a similar criterion it was registered the time of transition from cardiac arrhythmia to a persistent (> 5 sec.) ventricular tachycardia.

Aconitine induced arrhythmia in 100% of animals.

The results obtained are as follows :

| Treatments | Time of onset of initial arrhythmia (sec.) | Time of onset of initial tachycardia (sec.) |
|---|---|---|
| Saline, p.o. (control) | 155 ± 4 | 193 ± 8 |
| 50 mg/kg, p.o. | 163 ± 2 (P>0,05) | 245 ± 6 |
| 150 mg/kg, p.o. | 196 ± 10 | 286 ± 12 |
| Saline, i.p. (control) | 148 ± 5 | 186 ± 7 |
| 50 mg/kg, i.p. | 182 ± 7 | 264 ± 14 |
| 150 mg/kg, i.p. | 203 ± 12 | 298 ± 15 |

In conclusion, the slow infusion of aconitine to the anaesthetized mouse induces cardiac arrhythmia and ventricular tachycardia : the treatment with the formulation under study is able to prolong the onset time of such pathological forms generally in a statistical way.

A similar finding has been obtained by using as the end point the time of cardiac arrest even though the data are relatively more dispersed.

### Example 9

It has been adopted a technique similar to that described in Example 8 with the difference however that the treatment has been carried out in therapeutic form soon after the administration of reduced dosages of aconitine (0.5 mcg/ml, 0.25 ml/min.) in the anaesthetized mouse.

The composition of Table 5 in the form of sodium salt, was injected into the tail vein at doses of 50 and 100 mg/kg, determining in this case, because of the variability of the onset time of deviation from the normal sinus rhythm, just the appearance of persisting (> 5 sec.) arrhythmic forms respect to the total number of animals per group.

| | | | |
|---|---|---|---|
| | Controls (saline, i.v.) | 50 mg/kg, i.v. | 100 mg/kg, i.v. |
| Onset of arrhythmia | 9/10 | 3/10 | 1/10 |

### Example 10

A. The composition of Table 6 has been evaluated, in comparison with a mixture of saturated fats, for its ability to prevent or subdue the cardiac arrhythmia induced in the rat by an ischemic situation provoked by coronary ligation without use of arrhythmogenic agents. This experimental model appears to be more comparable to the similar ischemic or pre-infarction human pathology of atherosclerotic origin or otherwise arising.
   2 groups of 20 rats weighing 280 g ± 15 were fed with a standard laboratory diet containing 22% of protein and 15% of saturated fats. During this time one group of animals was also treated by oral route with 100 mg/kg daily of the composition of Table 6.
   At the end of treatment, the rats of the two groups were anaesthetized with sodium pentobarbital (50 mg/kg i.p.), inducing then an acute myocardial ischemia by surgical occlusion (ligation) of the left anterior descending coronary artery which permits the blood supply to the majority of the left ventricle. 2 animals per group dead during the surgical operation. The ischemia provoked by the coronary ligation was maintained for 15 minutes checking during this time the onset of arrhythmia and monitoring by ECG the parameters related to the premature ventricular beats (PVB), ventricular tachycardia (VT) and ventricular fibrillation (VF), which could be self terminating spontaneously or were also fatal with sudden death. The ventricular tachycardia was evidenced by several consecutive beats without a distinguishable P wave, the ventricular fibrillation was identified as a chaotic electric activity at the ECG control coupled with a sudden fall of the blood pressure. The results obtained are reported in the following table. 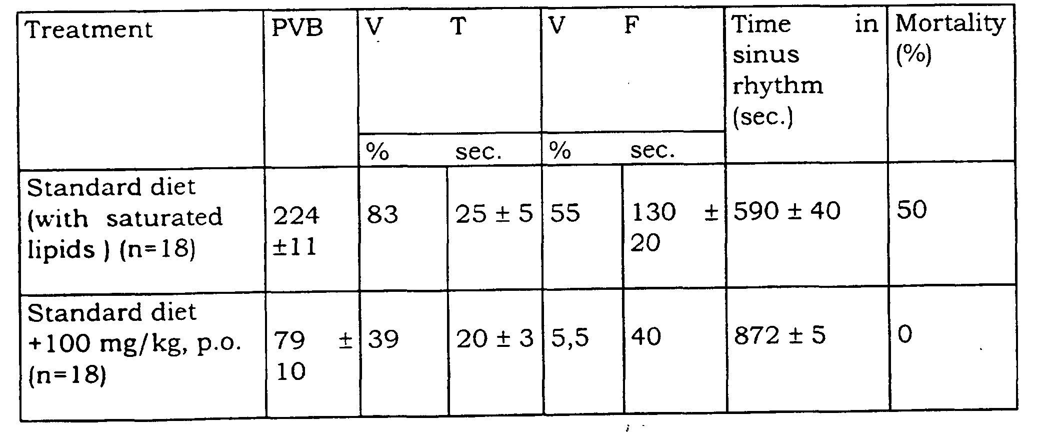
   The treatment with the composition under study has been shown therefore able to reduce clearly and in a statistically significant way the arrhythmia induced in the rat by experimental ischemia as shown by all the examined parameters (premature beats, tachycardia and ventricular fibrillation). In the described trial also the consequent mortality resulted to be avoided.
B. In the survived animals (controls, n=9; treated, n =18) the experiment was further continued by releasing the occluding ligation in order to restore the blood flow (reperfusion). After ECG control for 10 minutes, all the surviving rats were finally sacrificed . The purpose of the test was that of controlling the additional foreseeable episodes of arrhythmia which is known to be provoked also by a situation of ventricular reperfusion. This situation may be clinically related to post-ischemic and post-infarction situations, to coronary reperfusion following angioplasty etc.

For the above purpose it was once more monitored by means of ECG the premature ventricular beats (PVB), the ventricular tachycardia (VT) and the ventricular fibrillation (VF), with the results reported here below.

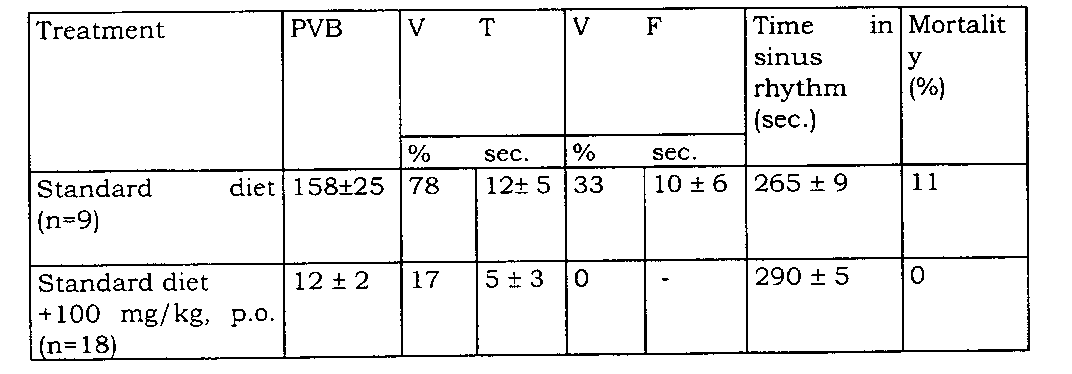

As it is evident, the restoring of the blood flow after the ischemic period, provoked the quick onset of arrhythmic episodes, including ventricular fibrillation, however less severe in degree and fatal to just one animal.

Even here however the animals treated with the composition of Table 6 were subject to a lower number of arrhythmic episodes, for a more limited time and with a survival of all the animals.

### Example 11

### Preparation of 1 g soft gelatine capsules

### Composition

- Ethyl esters of polyunsaturated fatty acids
   (EPA+DHA >85%; EPA/DHA = 0.9-1.5;
   other ω-3 C₂₀, C₂₁ and C₂₂, total < 3%) mg 1000
- D,L-α-tocoferol mg 0.3
- Gelatine succinate mg 233
- Glycerol mg 67
- Sodium ethyl p-hydroxibenzoate mg 1.09
- Sodium propyl p-hydroxibenzoate mg 0.54
Total weight about mg 1300

### Preparation

The composition of esters of fatty acids and the excipients are weighed and homogenized in a tank with a high speed stirrer.
The mixture is then treated with a colloidal mill and de-aerated in a stainless steel container. Proceed then to the inclusion in soft gelatine capsules by using conventional equipment and processes.

## Claims

1. Composition of fatty acids containing at least 80% by weight of acid 5,8,11,14,17-eicosapentaenoic acid (EPA) C₂₀ : 5 ω-3 (double bonds all cis) and acid 4,7,10,13,16,19-docosaexaenoic acid (DHA) C_{22:6} ω-3 (double bonds all cis), where the ratio EPA:DHA is within 0.9 and 1.5 and other ω-3 acids C₂₀, C₂₁ and C₂₂ constitute less than 3% and where such acids may be present in the form of free acids or their salts or short claim C₁-C₃ alkyl esters.

2. Composition according to claim 1, containing at least 85% by weight of EPA and DHA.

3. Composition according to claim 1, containing at least 90% by weight of EPA and DHA.

4. Composition according to claim 1, containing at least 95% by weight of EPA and DHA.

5. Composition according to claims 1-4, where the ratio EPA:DHA is within 1.0 and 1.4.

6. Composition according to claims 1-5 where the other ω-3 acids C₂₀,C₂₁ and C₂₂ constituting less than 3 % are represented by the acid C_{20:4} and/or C_{21:5} and/or C_{22:5} and eventual other acids in traces.

7. Composition according to claims 1-6 where the pharmaceutically acceptable salts are represented by salts with inorganic and organic bases.

8. Composition according to claims 1-7 where the alkyl esters are represented by the ethyl esters.

9. Composition according to claims 1-8 for treatment and/or prophylaxis of multiple risk factors for cardiovascular diseases and/or of cardiovascular diseases.

10. Procedure for production of a composition according to claims 1-9, where a crude oily material of marine origin is submitted to the following working steps in facultative sequence : hydrolysis or alcoholysis (transesterification), concentration and distillation by which a main fraction of composition according to claim 1 is isolated, each working step being done in conditions to avoid oxidation and isomerization of fatty acids; then, if desired, the acids obtained by hydrolysis are esterified or the esters obtained by alcoholysis are hydrolysed to free fatty acids and optionally transformed into salts or other derivatives pharmaceutically acceptable.

11. Procedure according to claim 10 where the crude oily material of marine origin is fish oil.

12. Procedure according to claims 10 or 11, where the fish oil has a content particularly high in EPA, preferably >12%, and in DHA, preferably >8% , and content in other ω-3 fatty acids C₂₀, C₂₁ and C₂₂ particularly low, preferably < 4% in total.

13. Procedure according to claims 10-12, where the fish oil has a content in C₂₂:5 ω-3 < 2.5% and a content in C_{21:5} ω-3 and C_{20:4} ω-3 <1% each.

14. Procedure according to claim 10, where the hydrolysis is carried out with alkalies in a mainly aqueous medium and alcoholysis (or transesterification) is carried out by alkaline or acid catalysis in a mainly alcoholic medium , preferably in ethanol.

15. Procedure according to claim 10, where the step of concentration is carried out through fractioning with urea.

16. Procedure according to claims 10 and 15, where the fractioning with urea is carried out in several working steps, initially by complexing with urea prevalently the saturated and mono-unsaturated fatty acids, and then prevalently the less-unsaturated, polyunsaturated fatty acids.

17. Procedure according to claim 10 where the distillation is carried out under normal pressure, under steam flow, under vacuum, under high vacuum and preferably with a molecular distillation technique, and preferably with a double stage molecular distillation.

18. Procedure according to claims 10 and 17, where the molecular distillation is carried out in order to isolate a fraction in agreement with the composition of claim 1 and to discard a fraction rich in other ω-3 acids C₂₀, C₂₁ and C₂₂ different from EPA and DHA.

19. Use of a composition according to claims 1-9 for the production of a pharmaceutical preparation for the treatment and/or the prophylaxis of multiple risk factors for cardiovascular diseases and/or cardiovascular diseases.

20. Use of a composition according to claims 1-9 and 19 for the production of a pharmaceutical preparation for the treatment and/or the prophylaxis of multiple risk factors represented by hypertriglyceridemia, hypercholesterolemia, hypertension and hyperactivity of factor VII of blood coagulation.

21. Use of a composition according to claims 1-9 and 19 for the production of a pharmaceutical preparation for the treatment and/or the prophylaxis of cardiovascular diseases deriving from familial hypertriglyceridemia and hypercholesterolemia.

22. Use of a composition according to claims 1-9 and 19 for the production of a pharmaceutical preparation for the treatment and/or the prophylaxis of cardiovascular diseases represented by atrial, and/or ventricular arrhythmia, tachycardia and/or fibrillation, and /or by defects of electric conduction of myocardial cells.

23. Use of a composition according to claims 1-9 and 22 for the production of a pharmaceutical preparation for the treatment and/or the primary or secondary prevention of cardiovascular diseases deriving from situations as described in claim 22.

24. Use of a preparation according to claims 1-9 and 23 for the production of a pharmaceutical preparation for the secondary prevention of cardiovascular diseases deriving from arrhythmia, tachycardia and/or fibrillation and/or defects of electric conduction induced by myocardial infarction.

25. Use of a composition according to claims 1-9 and 24 for the production of a pharmaceutical preparation for the prevention of myocardial re-infarction.

26. Use of a composition according to claims 1-9 and 24 for the production of a pharmaceutical preparation for the primary and secondary prevention of "sudden death" deriving from arrhythmia, tachycardia and/or fibrillation and/or defects of electric conduction of myocardial cells.

27. Procedure for production of a pharmaceutical preparation according to claims 19-26 comprising the inclusion in a pharmaceutically acceptable vehicle or excipient or diluent of a composition according to anyone of claims 1-9.

28. Pharmaceutical preparation according to claims 19-27 containing a composition of fatty acids according to anyone of claims 1-9.

29. Pharmaceutical preparation according to claim 28 for oral and parenteral use.

30. Pharmaceutical preparation according to claim 28, constituted by vials ready to use or by bottles for slow infusion or by a lyophilate for extemporary use, to be used by i.m. or i.v. route, and containing a composition of fatty acids according to anyone of claims 1-10 and where the vehicle is watery, oily or constituted by an emulsion.

31. Pharmaceutical preparation according to claim 28, constituted by soft gelatine capsules.

32. Pharmaceutical preparation according to claim 31 where the soft gelatine capsules contain from 250 to 1500 mg of a composition of fatty acids according to anyone of claims 1-9, preferably 500-1000 mg.

33. Use of a pharmaceutical preparation according to claims 28-32 for the preparation of a medicament for the prophylaxis and the treatment of atherosclerosis, defects of metabolism, autoimmune diseases, ulcerous colitis, tumor diseases, defects of learning or of memory and of central nervous system (CNS), processes of cell ageing, retinopathy, defect of platelet aggregation and of coagulation, brain infarction, cardiac and cerebral ischemic states and psoriasis.

## Patentansprüche

1. Fettsäurezusammensetzung, enthaltend wenigstens 80 Gew.-% der Säure 5, 8, 11, 14, 17-Eicosapentaensäure (EPA), C_{20:5}-ω-3, (Doppelbindungen alle cis) und der Säure 4,7,10,13,16,19-Docosahexaensäure (DHA), C_{22:6}-ω-3, (alle Doppelbindungen cis), wobei das Verhältnis EPA:DHA innerhalb des Bereichs von 0, 9 bis 1,5 liegt und andere ω-3-C₂₀-, -C₂₁- und -C₂₂-Säuren weniger als 3% ausmachen und wobei solche Säuren in Form von freien Säuren oder ihren Salzen oder kurzkettigen C₁-C₃-Alkylestern vorliegen können.

2. Zusammensetzung nach Anspruch 1, die wenigstens 85 Gew.-% EPA und DHA enthält.

3. Zusammensetzung nach Anspruch 1, die wenigstens 90 Gew.-% EPA und DHA enthält.

4. Zusammensetzung nach Anspruch 1, die wenigstens 95 Gew.-% EPA und DHA enthält.

5. Zusammensetzung nach den Ansprüchen 1 bis 4, wobei das Verhältnis EPA:DHA innerhalb des Bereichs von 1,0 bis 1,4 liegt.

6. Zusammensetzung nach den Ansprüchen 1 bis 5, wobei die anderen ω-3-C₂₀-, -C₂₁- und -C₂₂-Säuren, die weniger als 3% ausmachen, durch die Säure C_{20:4} und/oder C_{21:5} und/oder C_{22:5} und gegebenenfalls andere Säuren in Spuren dargestellt werden.

7. Zusammensetzung nach den Ansprüchen 1 bis 6, wobei die pharmazeutisch annehmbaren Salze durch Salze mit anorganischen oder organischen Basen dargestellt werden.

8. Zusammensetzung nach den Ansprüchen 1-7, wobei die Alkylester durch die Ethylester dargestellt werden.

9. Zusammensetzung nach den Ansprüchen 1-8 zur Behandlung und/oder Prophylaxe von mehreren Risikofaktoren für cardiovaskuläre Erkrankungen und/oder von cardiovaskulären Erkrankungen.

10. Verfahren zur Herstellung einer Zusammensetzung nach den Ansprüchen 1-9, wobei ein rohes öliges Material mariner Herkunft den folgenden Arbeitsschritten in falkutativer Folge unterworfen wird: Hydrolyse oder Alkoholyse (Umesterung), Konzentrierung und Destillation, durch welche eine Hauptfraktion der Zusammensetzung nach Anspruch 1 isoliert wird, wobei jeder Arbeitsschritt unter Bedingungen zur Vermeidung von Oxidation und Isomerisierung von Fettsäuren durchgeführt wird; danach, wenn es gewünscht wird, werden die durch Hydrolyse erhaltenen Säuren verestert oder die durch Alkoholyse erhaltenen Ester werden zu freien Fettsäuren hydrolysiert und gegebenenfalls in Salze oder andere Derivate, die pharmazeutisch annehmbar sind, überführt.

11. Verfahren nach Anspruch 10, wobei das rohe, ölige Material mariner Herkunft Fischöl ist.

12. Verfahren nach Anspruch 10 oder 11, wobei das Fischöl einen besonders hohen Gehalt an EPA, vorzugsweise >12%, und an DHA, vorzugsweise >8%, hat und einen besonders niedrigen Gehalt an anderen ω-3-C₂₀-, -C₂₁- und -C₂₂-Fettsäuren, vorzugsweise insgesamt <4%, hat.

13. Verfahren nach den Ansprüchen 10-12, wobei das Fischöl einen Gehalt an C_{22:5}-ω-3 <2,5% und einen Gehalt an C_{21:5}-ω-3 und C_{20:4}-ω-3 von je <1% hat.

14. Verfahren nach Anspruch 10, wobei die Hydrolyse mit Alkalien in einem hauptsächlich wässrigen Medium durchgeführt wird und eine Alkoholyse (oder Umesterung) durch alkalische oder saure Katalyse in einem hauptsächlich alkoholischen Medium, vorzugsweise in Ethanol, durchgeführt wird.

15. Verfahren nach Anspruch 10, wobei der Konzentrierungsschritt durch Fraktionierung mit Harnstoff durchgeführt wird.

16. Verfahren nach Anspruch 10 und 15, wobei die Fraktionierung mit Harnstoff in mehreren Arbeitsschritten durchgeführt wird, zunächst durch Komplexbildung vorwiegend der gesättigten und einfach ungesättigten Fettsäuren und dann vornehmlich der weniger ungesättigten, mehrfach ungesättigten Fettsäuren.

17. Verfahren nach Anspruch 10, wobei die Destillation unter Normaldruck, unter Dampfstrom, unter Vakuum, unter Hochvakuum und vorzugsweise mit einer Molekulardestillationstechnik und vorzugsweise mit einer Zweistufenmolekulardestillation durchgeführt wird.

18. Verfahren nach Anspruch 10 und 17, wobei die Molekulardestillation durchgeführt wird, um eine Fraktion entsprechend der Zusammensetzung nach Anspruch 1 zu isolieren und eine Fraktion, die reich an anderen ω-3-C₂₀-, -C₂₁- und -C₂₂-Säuren als EPA und DHA ist, zu verwerfen.

19. Verwendung einer Zusammensetzung nach den Ansprüchen 1-9 zur Herstellung eines pharmazeutischen Präparats zur Behandlung und/oder Prophylaxe von mehreren Risikofaktoren für cardiovaskuläre Erkrankungen und/oder von cardiovaskulären Erkrankungen.

20. Verwendung einer Zusammensetzung nach den Ansprüchen 1-9 und 19 zur Herstellung eines pharmazeutischen Präparats für die Behandlung und/oder die Prophylaxe von mehreren Risikofaktoren, die durch Hypertriglyceridämie, Hypercholesterinämie, Bluthochdruck und Hyperaktivität von Faktor VII der Blutgerinnung dargestellt werden.

21. Verwendung einer Zusammensetzung nach den Ansprüchen 1-9 und 19 zur Herstellung eines pharmazeutischen Präparats zur Behandlung und/oder Prophylaxe von cardiovaskulären Erkrankungen, die sich von familiärer Hypertriglyceridämie und Hypercholesterinämie ableiten.

22. Verwendung einer Zusammensetzung nach den Ansprüchen 1-9 und 19 zur Herstellung eines pharmazeutischen Präparats für die Behandlung und/oder die Prophylaxe von cardiovaskulären Erkrankungen, die durch Vorhofflimmern und/oder Kammerflimmern, Tachycardie und/oder Fibrillation und/oder durch Fehler der elektrischen Leitung von myocardialen Zellen dargestellt werden.

23. Verwendung einer Zusammensetzung nach den Ansprüchen 1-9 und 22 zur Herstellung eines pharmazeutischen Präparats für die Behandlung und/oder die Primär- oder Sekundärprävention von cardiovaskulären Erkrankungen, die sich aus Situationen ableiten, wie sie in Anspruch 22 beschrieben sind.

24. Verwendung eines Präparats nach den Ansprüchen 1-9 und 23 zur Herstellung eines pharmazeutischen Präparats für die Sekundärprävention von cardiovaskulären Erkrankungen, die sich von Arrhythmie, Tachycardie und/oder Fibrillation und/oder Fehlern der elektrischen Leitung, induziert durch Myocardinfarkt, ableiten.

25. Verwendung einer Zusammensetzung nach den Ansprüchen 1-9 und 24 zur Herstellung eines pharmazeutischen Präparats für die Prävention eines Myocardreinfarkts.

26. Verwendung einer Zusammensetzung nach den Ansprüchen 1-9 und 24 zur Herstellung eines pharmazeutischen Präparats für die Primär- und Sekundärprävention von "plötzlichem Tod", der aus Arrhythmie, Tachycardie und/oder Fibrillation und/oder Fehlern der elektrischen Leitung von myocardialen Zellen resultiert.

27. Verfahren zur Herstellung eines pharmazeutischen Präparats nach den Ansprüchen 19-26, das den Einschluss einer Zusammensetzung nach einem der Ansprüche 1-9 in einem pharmazeutisch akzeptablen Träger oder Exzipienten oder Verdünnungsmittel umfasst.

28. Pharmazeutisches Präparat nach den Ansprüchen 19-27, das eine Fettsäurezusammensetzung nach einem der Ansprüche 1-9 enthält.

29. Pharmazeutisches Präparat nach Anspruch 28 zur oralen und parenteralen Verwendung.

30. Pharmazeutisches Präparat nach Anspruch 28, das aus gebrauchsfertigen Phiolen oder Flaschen zur langsamen Infusion oder aus Lyophilisat zur extemporären Verwendung, um auf i.m.- oder i.v.-Weg verwendet zu werden, besteht und eine Fettsäurezusammensetzung nach einem der Ansprüche 1-10 enthält und wobei das Vehikel wässrig, ölig ist oder durch eine Emulsion gebildet wird.

31. Pharmazeutisches Präparat nach Anspruch 28, das aus weichen Gelatinekapseln besteht.

32. Pharmazeutisches Präparat nach Anspruch 31, wobei die weichen Gelatinekapseln 250 bis 1500 mg einer Fettsäurezusammensetzung nach einem der Ansprüche 1-9, vorzugsweise 500-1000 mg, enthalten.

33. Verwendung eines pharmazeutischen Präparats nach den Ansprüchen 28-32 zur Herstellung eines Medikaments für die Prophylaxe und Behandlung von Atherosklerose, Stoffwechselstörungen, Autoimmunerkrankungen, Colitis ulcerosa, Tumorerkrankungen, Lernstörungen oder Störungen des Gedächtnisses und des zentralen Nervensystems (ZNS), Prozessen der Zellalterung, Retinopathie, Defekten der Thrombozytenaggregation und der Coagulation, Gehirnschlag, cardialen und zerebralen ischämischen Zuständen und Psoriasis.

## Revendications

1. Composition d'acides gras contenant au moins 80 % en poids d'acide 5,8,11,14,17-éicosapentaénoïque (EPA) C_{20:5} ω-3 (toutes les doubles liaisons cis) et d'acide 4,7,10,13,16,19-docosaéxaénoïque (DHA) C₂₂₋₆ ω-3 (toutes les doubles liaisons cis), dans laquelle le ratio EPA/DHA est entre 0,9 et 1,5 et d'autres acides ω-3 C₂₀, C₂₁ et C₂₂ constituent moins d'environ 3 % et dans laquelle ces acides peuvent être présents sous la forme d'acides libres ou de leurs sels ou d'esters d'alkyle C₁-C₃ à chaîne courte.

2. Composition selon la revendication 1, contenant au moins 85 % en poids d'EPA et de DHA.

3. Composition selon la revendication 1, contenant au moins 90 % en poids d'EPA et de DHA.

4. Composition selon la revendication 1, contenant au moins 95% en poids d'EPA et de DHA.

5. Composition selon les revendications 1-4, dans laquelle le ratio EPA/DHA est entre 1,0 et 1,4.

6. Composition selon les revendications 1-5, dans laquelle les autres acides ω-3 C₂₀, C₂₁ et C₂₂ constituant moins de 3 %, sont réprimés par l'acide C_{20:4} et/ou C_{21:5} et/ou C_{22:5} et d'autres acides éventuels sous forme de traces.

7. Composition selon les revendications 1-6 dans laquelle les sels pharmaceutiquement acceptables sont représentés par des sels avec des bases inorganiques et organiques.

8. Composition selon les revendications 1-7 dans laquelle les esters d'alkyle sont représentés par les esters d'éthyle.

9. Composition selon les revendications 1-8 pour le traitement et/ou la prophylaxie de multiples facteurs de risque de maladies cardio-vasculaires et/ou de maladies cardio-vasculaires.

10. Procédure pour la production d'une composition selon les revendications 1-9, dans laquelle un matériau huileux brut d'origine marine est soumis aux étapes de travail suivantes en séquence facultative : hydrolyse ou alcoolyse (transestérification), concentration et distillation par lesquelles une fraction principale de la composition selon la revendication 1 est isolée, chaque étape de travail étant réalisée dans des conditions permettant d'éviter l'oxydation et l'isomérisation des acides gras ; ensuite, si souhaité, les acides obtenus par hydrolyse sont estérifiés ou les esters obtenus par alcoolyse sont hydrolysés pour libérer les acides gras et facultativement transformés en sels ou autres dérivés pharmaceutiquement acceptables.

11. Procédure selon la revendication 10 dans laquelle le matériau huileux brut d'origine marine est de l'huile de poisson.

12. Procédure selon la revendication 10 ou 11 dans laquelle l'huile de poisson a une teneur particulièrement élevée en EPA, de préférence supérieure à 12 %, et en DHA, de préférence supérieure à 8 % et une teneur en autres acides gras ω-3 C₂₀, C₂₁ et C₂₂ particulièrement faible, de préférence inférieure à 4 % au total.

13. Procédure selon les revendications 10 à 12, dans laquelle l'huile de poisson a une teneur en C_{22:5} ω-3 inférieure à 2,5 % et une teneur en C_{21:5} ω-3 et en C_{20:4} ω-3 inférieures à 1 % chacune.

14. Procédure selon la revendication 10, dans laquelle l'hydrolyse est réalisée avec des alcalis dans un milieu principalement aqueux et l'alcoolyse (transestérification) est réalisée par catalyse alcaline ou acide dans un milieu principalement alcoolique, de préférence dans de l'éthanol.

15. Procédure selon la revendication 10, dans laquelle l'étape de concentration est réalisée par fractionnement avec de l'urée.

16. Procédure selon les revendications 10 et 15, dans lesquelles le fractionnement avec de l'urée est réalisé en plusieurs étapes de travail, initialement par mise en complexe avec de l'urée de manière prévalente des acides gras saturés et mono-insaturés, puis de manière prévalente les acides gras moins insaturés, polyinsaturés.

17. Procédure selon la revendication 10 dans laquelle la distillation est réalisée sous une pression normale, sous un flux de vapeur, sous vide, sous vide élevé et de préférence avec une technique de distillation moléculaire, et de préférence avec une distillation moléculaire en deux étapes.

18. Procédure selon les revendications 10 et 17, dans laquelle la distillation moléculaire est réalisée afin d'isoler une fraction en accord avec la composition de la revendication 1 et afin d'éliminer une fraction riche en d'autres acides ω-3 C₂₀, C₂₁ et C₂₂ différents de l'EPA et du DHA.

19. Utilisation d'une composition selon les revendications 1 à 9 pour la production d'une préparation pharmaceutique pour le traitement et/ou la prophylaxie de facteurs de risques multiples de maladies cardio-vasculaires et/ou de maladies cardio-vasculaires.

20. Utilisation d'une composition selon les revendications 1-9 et 19 pour la production d'une préparation pharmaceutique pour le traitement et/ou la prophylaxie de facteurs de risque multiples représentés par l'hypertriglycéridémie, l'hypercholestérolémie, l'hypertension et l'hyperactivité du facteur VII de la coagulation sanguine.

21. Utilisation d'une composition selon les revendications 1-9 et 19 pour la production d'une préparation pharmaceutique pour le traitement et/ou la prophylaxie de maladies cardio-vasculaires dérivant de l'hypertriglycéridémie familiale et de l'hypercholestérolémie.

22. Utilisation d'une composition selon les revendications 1-9 et 19 pour la production d'une préparation pharmaceutique pour le traitement et/ou la prophylaxie de maladies cardio-vasculaires représentées par l'arythmie auriculaire et/ou ventriculaire, la tachycardie et/ou la fibrillation, et/ou par des défauts de la conduction électrique des cellules du myocarde.

23. Utilisation d'une composition selon les revendications 1-9 et 22 pour la production d'une préparation pharmaceutique pour le traitement et/ou la prévention primaire ou secondaire de maladies cardiovasculaires dérivant de situations telles celles décrites dans la revendication 22.

24. Utilisation d'une préparation selon les revendications 1-9 et 23 pour la production d'une préparation pharmaceutique pour la prévention secondaire de maladies cardio-vasculaires dérivant de l'arythmie, de la tachycardie et/ou de la fibrillation et/ou de défauts de la conduction électrique induits par l'infarctus du myocarde.

25. Utilisation d'une composition selon les revendications 1-9 et 24 pour la production d'une préparation pharmaceutique pour la prévention de la récidive de l'infarctus du myocarde.

26. Utilisation d'une composition selon les revendications 1-9 et 24 pour la production d'une préparation pharmaceutique pour la prévention primaire et secondaire de la « mort subite » dérivant de l'arythmie, de la tachycardie et/ou de la fibrillation et/ou de défauts de la conduction électrique induits par l'infarctus du myocarde.

27. Procédure pour la production d'une préparation pharmaceutique selon les revendications 19-26 comprenant l'inclusion dans un véhicule ou un excipient ou un diluant pharmaceutiquement acceptables d'une composition selon l'une quelconque des revendications 1-9.

28. Préparation pharmaceutique selon les revendications 19-27 contenant une composition d'acides gras selon l'une quelconque des revendications 1-9.

29. Préparation pharmaceutique selon la revendication 28 pour utilisation orale et parentérale.

30. Préparation pharmaceutique selon la revendication 28, constituée par des flacons prêts à l'emploi ou par des flacons pour perfusion lente ou par un lyophilisat pour utilisation extemporanée, à utiliser par voie IM ou IV, et contenant une composition d'acides gras selon l'une quelconque des revendications 1 à 10 et dans laquelle le véhicule est aqueux, huileux ou constitué d'une émulsion.

31. Préparation pharmaceutique selon la revendication 28, constituée par des capsules de gélatine molles.

32. Préparation pharmaceutique selon la revendication 31 dans laquelle les capsules de gélatine molles contiennent de 250 à 1 500 mg d'une composition d'acides gras selon l'une quelconque des revendications 1-9, de préférence 500 à 1 000 mg.

33. Utilisation d'une préparation pharmaceutique selon les revendications 28-32 pour la préparation d'un médicament pour la prophylaxie et le traitement de l'athérosclérose, de défauts du métabolisme, de maladies auto-immunes, de la rectocolite hémorragique, de maladies tumorales, de défauts de l'apprentissage ou de la mémoire et du système nerveux central (SNC), des processus de vieillissement cellulaire, de la rétinopathie, de défauts de l'agrégation plaquettaire et de la coagulation, d'infarctus cérébraux, d'états d'ischémie cardiaque et cérébrale et du psoriasis.
